(19)

Europäisches Patentamt
European Patent Office
Office européen des brevets

(11) **EP 3 667 673 A1**

(12) **EUROPEAN PATENT APPLICATION**

(43) Date of publication:
**17.06.2020 Bulletin 2020/25**

(51) Int Cl.:
**G16C 20/40** (2019.01)    **G16B 15/00** (2019.01)

(21) Application number: **19205295.9**

(22) Date of filing: **25.10.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(30) Priority: **03.12.2018  JP 2018226351** | (71) Applicant: **FUJITSU LIMITED**<br>**Kanagawa 211-8588 (JP)**<br><br>(72) Inventor: **SATO, Hiroyuki**<br>**Kawasaki-shi, Kanagawa 211-8588 (JP)**<br><br>(74) Representative: **Haseltine Lake Kempner LLP**<br>**Lincoln House, 5th Floor**<br>**300 High Holborn**<br>**London WC1V 7JH (GB)** |

(54) **METHOD AND DEVICE FOR SEARCHING STRUCTURE OF CYCLIC MOLECULE, AND PROGRAM**

(57)    A method includes arranging each of compound groups in the number of n on each of lattice points to create three-dimensional structure of cyclic molecule in three-dimensional lattice space where the lattice points are lattice points of the three-dimensional lattice space that is collection of lattices, the method being method for searching stable structure of the cyclic molecule where the compound groups are linked to form ring using computer, and wherein in case where the number (n) is odd number, the arranging includes: inserting linking group between the compound group arranged in the order of n and the compound group arranged first within the cyclic molecule, arranging the linking group on lattice point, and adjusting the arrangement in a manner that the compound group arranged in the order of n and the compound group arranged first do not face each other with the linking group being therebetween.

FIG. 6

S101 — Defining three-dimensional lattice space

S102 — Is the number (n) of amino acid residues odd number?

No → S103 — Adding linking group S and terminal group E

Yes

S104 — Determining collection of lattice points that are locations No. i amino acid residue moves as $V_i$

S105 — Assigning information of space to $X_1$ to $X_n$

S106 — Creating Ising model

S107 — Determining the minimum value of $E(x)$ by annealing machine

S108 — Outputting result

EP 3 667 673 A1

**Description**

FIELD

The embodiments discussed herein relate to a method and device for searching a stable structure of a cyclic molecule using a three-dimensional lattice space, and a program.

BACKGROUND

It is often a case that a stable structure of a large-size molecule is important. Examples of such a case include drug discovery. However, it may be difficult to search a stable structure of a large-size molecule, such as a protein, within a realistic time scale according a calculation clarifying and considering all of atoms.

Therefore, a technology for shortening a calculation time by roughly capturing (roughly visualizing) a structure of a molecule has been researched. For example, researched is a technology, which is called lattice protein, where a roughly visualized structure of a protein composed of only one-dimensional alignment information of amino acid residues is searched. As one example thereof, a technology for searching a stable structure of a simple cubic lattice structure of a straight-chain protein high speed using a technology of quantum annealing has been reported. According to the current technologies, the technology for searching a structure of a lattice protein by an annealing machine, such as quantum annealing, can be only applied to a straight-chain structure. Considering a drug candidate compound bonded to a protein serving as a target in the field of drug discovery, however, a cyclic compound is more strongly bonded than a straight-chain compound. In view of application to drug discovery, therefore, it is important that a search of stable structure of a cyclic molecule is made possible. One example of the background technology is disclosed in R. Babbush et.al., Construction of Energy Functions for Lattice Heteropolymer Models: A Case Study in Constraint Satisfaction Programming and Adiabatic Quantum Optimization, arXiv:quant-ph/1211.3422v2 (https://arxiv.org/abs/1211.3422).

SUMMARY

It is desirable to provide a method and device for searching a structure of a cyclic molecule, which can search a stable structure of a cyclic molecule, and a program for causing a computer to execute the method for searching a structure.

According to an embodiment of one aspect of the present disclosure, a method for searching a structure of a cyclic molecule is a method for searching a stable structure of the cyclic molecule, in which the compound groups in the number of n are linked to form a ring, using a computer. The method includes arranging each of compound groups in the number of n on each of lattice points to create a three-dimensional structure of the cyclic molecule in a three-dimensional lattice space, where the lattice points are lattice points of the three-dimensional lattice space that is a collection of lattices. In a case where the number (n) of the compound groups of the cyclic compound is an odd number, the arranging includes inserting a linking group between the compound group arranged in the order of n and the compound group arranged first within the cyclic molecule, arranging the linking group on a lattice point, and adjusting the arrangement in a manner that the compound group arranged in the order of n and the compound group arranged first do not face each other with the linking group being between the compound arranged in the order of n and the compound group arranged first.

According to an embodiment of another aspect of the present disclosure, a device for searching a structure of a cyclic molecule is a device for searching a stable structure of the cyclic molecule in which the compound groups in the number of n are linked to form a ring. The device includes a creating unit configured to arrange each of compounds in the number of n on each of lattice points to create a three-dimensional structure of the cyclic molecule in a three-dimensional lattice space, where the lattice points are lattice points of the three-dimensional lattice space that is a collection of lattices. In a case where the number (n) of the compound groups of the cyclic compound is an odd number, the creating unit is configured to insert a linking group between the compound group arranged in the order of n and the compound group arranged first within the cyclic molecule, arrange the linking group on a lattice point, and adjust the arrangement in a manner that the compound group arranged in the order of n and the compound group arranged first do not face each other with the linking group being between the compound arranged in the order of n and the compound group arranged first.

According to an embodiment of another aspect of the present disclosure, a program for causing a computer to execute a method for searching a structure of cyclic molecule is a program for searching a stable structure of the cyclic molecule in which the compound groups in the number of n are linked to form a ring. The method includes arranging each of compound groups in the number of n on each of lattice points to create a three-dimensional structure of the cyclic molecule in a three-dimensional lattice space, where the lattice points are lattice points of the three-dimensional lattice space that is a collection of lattices. In a case where the number (n) of the compound groups of the cyclic compound is an odd number, the arranging includes inserting a linking group between the compound group arranged in the order of n and the compound group arranged first within the cyclic molecule, arranging the linking group on a lattice point, and adjusting the arrangement in a manner that the compound group arranged in the order of n and the compound group arranged first do not face each other with the linking group being between the compound arranged in the order of n and the compound group arranged first.

The disclosed method for searching a structure of a cyclic molecule can solve the various problems existing in the art, and can provide a method for searching a structure of a cyclic molecule, which can search a stable structure of a cyclic molecule.

The disclosed device for searching a structure of a cyclic molecule can solve the various problems existing in the art, and can provide a device for searching a structure of a cyclic molecule, which can search a stable structure of a cyclic molecule.

The disclosed program can solve the various problems existing in the art, and can provide a program which can search a stable structure of a cyclic molecule.

BRIEF DESCRIPTION OF DRAWINGS

FIG. 1A is a schematic view for searching a stable structure of a protein (part 1).
FIG. 1B is a schematic view for searching a stable structure of a protein (part 2).
FIG. 1C is a schematic view for searching a stable structure of a protein (part 3).
FIG. 2A is a schematic view for describing the diamond encoding method (part 1).
FIG. 2B is a schematic view for describing the diamond encoding method (part 2).
FIG. 2C is a schematic view for describing the diamond encoding method (part 3).
FIG. 2D is a schematic view for describing the diamond encoding method (part 4).
FIG. 2E is a schematic view for describing the diamond encoding method (part 5).
FIG. 3A is a schematic view for illustrating an example of arrangement in case of a cyclic protein the number of amino acid residues of which is 8.
FIG. 3B is a schematic view for illustrating an example of arrangement in case of a cyclic protein the number of amino acid residues of which is 7.
FIG. 3C is a schematic view for illustrating another example of arrangement in case of a cyclic protein the number of amino acid residues of which is 7.
FIG. 4 is a schematic view illustrating an example where a linking group S is added in case of a cyclic protein the number of amino acid residues of which is 7.
FIG. 5 is a schematic view illustrating an example where a linking group S is added in case of a cyclic protein the number of amino acid residues of which is 7.
FIG. 6 is a flowchart illustrating a method for searching a stable structure of a protein.
FIG. 7 is a view illustrating a case where each lattice within a radius r is $S_r$.
FIG. 8A is a view illustrating a collection of lattice points to which an amino acid residue moves (part 1).
FIG. 8B is a view illustrating a collection of lattice points to which an amino acid residue moves (part 2).
FIG. 8C is a view illustrating a collection of lattice points to which an amino acid residue moves (part 3).
FIG. 8D is a view illustrating a collection of lattice points to which an amino acid residue moves (part 4).
FIG. 9 is a view representing $S_1$, $S_2$, and $S_3$ three-dimensionally.
FIG. 10A is a view illustrating an example of a state where information of space is assigned to each of bits $X_1$ to $X_n$ (part 1).
FIG. 10B is a view illustrating an example of a state where information of space is assigned to each of bits $X_1$ to $X_n$ (part 2).
FIG. 10C is a view illustrating an example of a state where information of space is assigned to each of bits $X_1$ to $X_n$ (part 3).
FIG. 11 is a view for describing $H_{one}$.
FIG. 12 is a view for describing $H_{conn}$.
FIG. 13 is a view for describing $H_{olap}$.
FIG. 14A is a view for describing $H_{pair}$ (part 1).
FIG. 14B is a view for describing $H_{pair}$ (part 2).
FIG. 15 is a diagram illustrating a weight file.
FIG. 16 is a view illustrating a conceptual structure of an optimizing device (arithmetic unit) used for simulated annealing.
FIG. 17 is a block diagram of a circuit level of a transition controlling unit.

FIG. 18 is a view illustrating an operation flow of a transition controlling unit.

FIG. 19 is a view illustrating a structural example of the disclosed device for searching a structure of a cyclic molecule.

FIG. 20 is a view illustrating another structural example of the disclosed device for searching a structure of a cyclic molecule.

FIG. 21 is a view illustrating another structural example of the disclosed device for searching a structure of a cyclic molecule.

DESCRIPTION OF EMBODIMENTS

The disclosed method for searching a structure of a cyclic molecule is a method for searching a stable structure of the cyclic molecule, in which the compound groups in the number of n are linked to form a ring, using a computer.

The method for searching a structure of a cyclic molecule includes a creating step, and may further include other steps according to the necessity.

The creating step includes arranging each of compound groups in the number of n on each of lattice points to create a three-dimensional structure of the cyclic molecule in a three-dimensional lattice space, where the lattice points are lattice points of the three-dimensional lattice space that is a collection of lattices.

In the case where the number (n) of the compound groups of the cyclic molecule is an odd number, the creating step includes the following processes 1 to 3.

Process 1: a process for inserting a linking group between the compound group arranged in the order of n and the compound group arranged first within the cyclic molecule.

Process 2: a process for arranging a linking group on a lattice point.

Process 3: a process for adjusting the arrangement in a manner that the compound group arranged in the order of n and the compound group arranged first do not face each other with the linking group being between the compound arranged in the order of n and the compound group arranged first.

Before describing the details of the disclosed technology, a method for determining folding of a protein according to the diamond encoding method will be described.

A search of a stable structure of a protein is typically performed in the following manner.

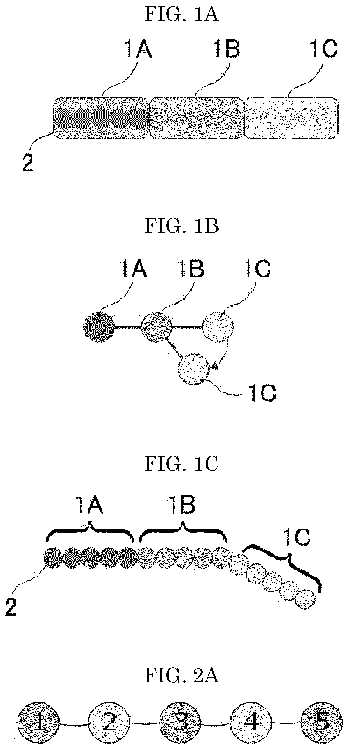

First, coarse graining of a protein is performed (FIG. 1A). For example, the coarse graining of a protein is performed by coarse graining atoms 2 constituting the proteins into amino acid residue units 1A, 1B, and 1C.

Next, a structure search is performed using the created coarse-grained model (FIG. 1B). The structure search is performed according to the diamond encoding method described later.

Next, the coarse-grained model is returned back to the whole atoms (FIG. 1C).

The diamond encoding method is generally a method where a linear amino acid is embedded in a position on a diamond lattice, and can represents a three-dimensional structure. For the sake of simplicity, a two-dimensional structure is described as an example.

Used as an example is a linear pentapeptide having a structure illustrated in FIG. 2A, where 5 amino acid residues are linked, when the structure is represented by a linear structure. In FIGs. 2A to 2E, a number in each circle is a number of the amino acid residue in the linear pentapeptide.

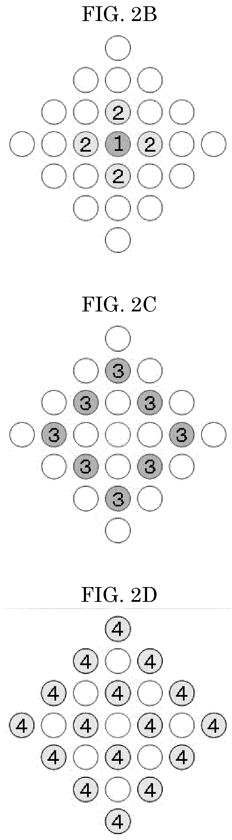

First, an amino acid residue of No. 1 is arranged at a center of a diamond lattice as illustrated in FIG. 2A, positions where an amino acid residue of No. 2 can be arranged are limited to positions next to the center as illustrated in FIG. 2B (the positions numbered as 2).

Next, in FIG. 2C, positions to which an amino acid residue of No. 3 bonded to next to the amino acid residues of No. 2 can be arranged are limited to positions next to the positions numbered as 2 (the positions numbered as 3) in FIG. 2B.

Next, in FIG. 2D, positions to which an amino acid residue of No. 4 bonded to next to the amino acid residues of No. 3 can be arranged are limited to positions next to the positions numbered as 3 (the positions numbered as 4) in FIG. 2C.

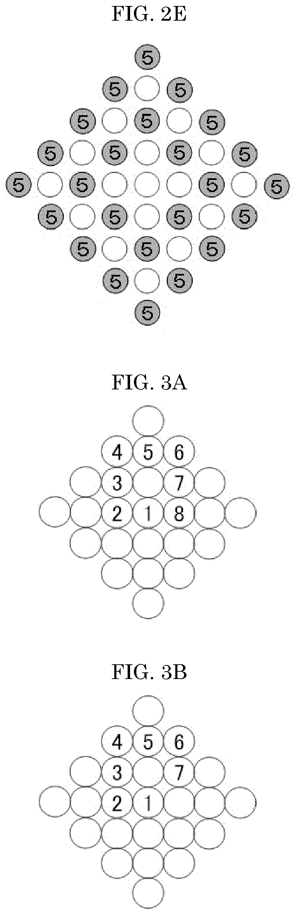

Next, in FIG. 2E, positions to which an amino acid residue of No. 5 bonded to next to the amino acid residues of No. 4 can be arranged are limited to positions next to the positions numbered as 4 (the positions numbered as 5) in FIG. 2D.

In the manner as described above, a three-dimensional structure can be expressed by linking the positions where amino acid residues can be arranged.

The above-described technology in the art is applied to a cyclic protein that is a cyclic molecule as follows.

As a case of a cyclic protein in which the number of amino acid residues is an even number, a cyclic protein the number of amino acid residues of which is 8 is described with reference to FIG. 3A. In case of a cyclic protein the number of amino acid residues of which is 8, the amino acid residue arranged at the first of the alignment (first) and the amino acid residue arranged at the 8th of the alignment (last) can be arranged on lattices next to each other, and therefore a cyclic structure can be reproduced in a diamond lattice.

As a case where a cyclic protein in which the number of amino acid residues is an odd number, a cyclic protein the number of amino acid residues of which is 7 is described with reference to FIG. 3B. In case of a cyclic protein the number of amino acid residues of which is 7, the amino acid residue arranged at the first of the alignment (first) and the amino acid residue arranged at the 7th of the alignment (last) cannot be arranged on lattices next to each other, and therefore a cyclic structure cannot be reproduced in a diamond lattice.

In case of a cyclic protein in which the number of amino acid residues is an odd number, therefore, a three-dimensional structure cannot be obtained.

In the case as illustrated in FIG. 3B, however, the amino acid residue arranged at the first of the alignment (first) and the amino acid residue arranged at the 7th of the alignment (last) are closely arranged from each other, and therefore the arrangement can be considered as a realizable cyclic structure.

Therefore, the present inventors researched a method for obtaining a three-dimensional structure of a cyclic protein in a case as illustrated in FIG. 3B.

Since the amino acid residue arranged at the first of the alignment (first) and the amino acid residue arranged at the 7th of the alignment (last) are present close to each other in FIG. 3B, a cyclic protein can be obtained by performing a process for linking between the amino acid residue arranged first and the amino acid residue arranged last.

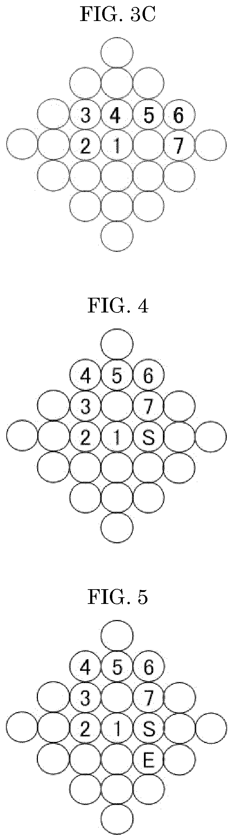

In case of the alignment as illustrated in FIG. 3C, meanwhile, it is not appropriate to link between the amino acid residue arranged at the first of the alignment (first) and the amino acid residue arranged at the 7th of the alignment (last) because a distance between the amino acid residue arranged first and the amino acid residue arranged last is large.

Therefore, the present inventors have the solved the above-described matter as follows. In case of a cyclic protein in which the number of amino acid residues is an odd number, as illustrated in FIG. 4, a linking group S is added and the compound group arranged last and the compound group arranged first do not face each other with the linking group being between the compound group arranged last and the compound group arranged last.

As a result, the lattice points are connected in the form of a ring with the linking group S to thereby obtain a three-dimensional structure of the cyclic protein in a diamond lattice. Moreover, it can avoid to make a distance between the amino acid residue arranged at the first of the alignment (first) and the amino acid residue arranged at the 7th of the alignment (last) large by arranging in the manner that the compound group arranged last and the compound group arranged first are not face to each other with the linking group being between the compound group arranged last and the compound group arranged first, as illustrated in FIG. 3C.

The present inventors have found that a stable structure of a cyclic molecule can be obtained as follows according to a method for searching a stable structure of a cyclic molecule in which compound groups in the number of n are linked to form a ring. Specifically, each of the compound groups in the number of n is arranged on each of lattice points to create a three-dimensional structure of the cyclic molecule in a three-dimensional lattice space, where the lattice points are lattice points of the three-dimensional lattice space that is a collection of lattices. In the case where the number (n) of the compound groups of the cyclic molecule is an odd number, performed to determine a stable structure of the cyclic molecule are inserting a linking group between a compound group arranged in the order of n and a compound group arranged first within the cyclic molecule, arranging the linking group on a lattice point, and adjusting the arrangement in a manner that the compound group arranged in the order of n and the compound group arranged first do not face each other with the linking group being between the compound arranged in the order of n and the compound group arranged first.

The adjusting the arrangement is preferably performed by bonding a terminal group to the linking group to arrange the terminal group to face the compound group arranged in the order of n in the three-dimensional lattice space with the linking group being between the terminal group and the compound group.

As illustrated in FIG. 5, for example, a terminal group E is added to a position facing the amino acid residue arranged at the 7th of the alignment (last) with the linking group between the terminal group and the amino acid residue arranged last. As a result of the arrangement as described, it can avoid to make a distance between the amino acid residue arranged at the first of the alignment (first) and the amino acid residue arranged at the 7th of the alignment (last) large in the obtained cyclic molecule, as illustrated in FIG. 3C.

Note that, the linking group and the terminal group are imaginary groups that are not actually present in the cyclic molecule.

Moreover, a ground state search is performed on the created three-dimensional structure of the cyclic molecule using simulated annealing to thereby calculate the minimum energy.

For example, the compound groups are amino acid residues.

In the case where the compound groups are amino acid residues, examples of the cyclic molecule include a cyclic protein.

Amino acid that is a base of an amino acid residue may be natural amino acid or synthetic amino acid. Examples of the natural amino acid include alanine, arginine, asparagine, aspartic acid, cysteine, glutamine, glutamic acid, glycine, histidine, isoleucine, leucine, lysine, methionine, phenyl alanine, proline, serine, threonine, tryptophan, tyrosine, valine, β-alanine, and β-phenylalanine. Examples of the synthetic amino acid include para-benzoylphenylalanine.

The number of amino acid residues in the protein is not particularly limited and may be appropriately selected depending on the intended purpose. For example, the number thereof may be from about 10 to about 30, or about several hundreds.

One example of the disclosed technology will be described using a flowchart etc. hereinafter.

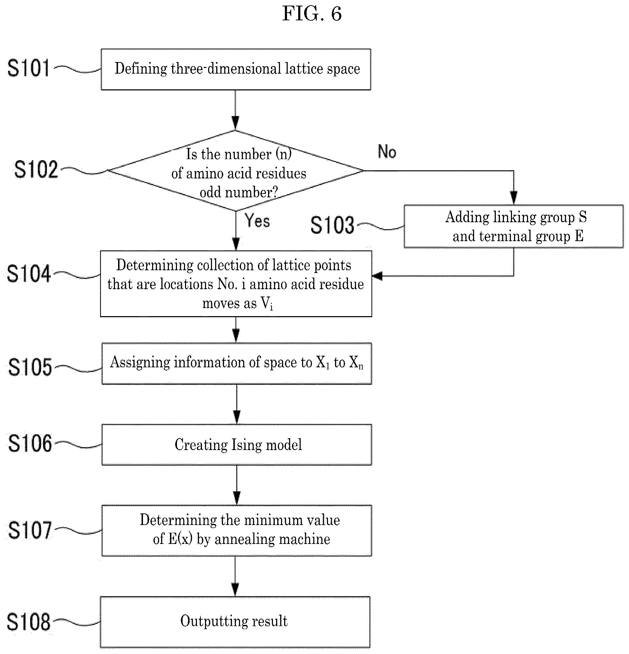

FIG. 6 is a flowchart for searching a stable structure of a protein.

<Step S101>

First, a three-dimensional lattice space that is a collection of lattices to which a plurality of the amino acid residues are arranged is defined based on the number (n) of the amino acid residues (S101).

One example of the definition of the three-dimensional lattice space will be described. The lattice space is three dimensional, but a two dimensional lattice space is described as an example for simplicity.

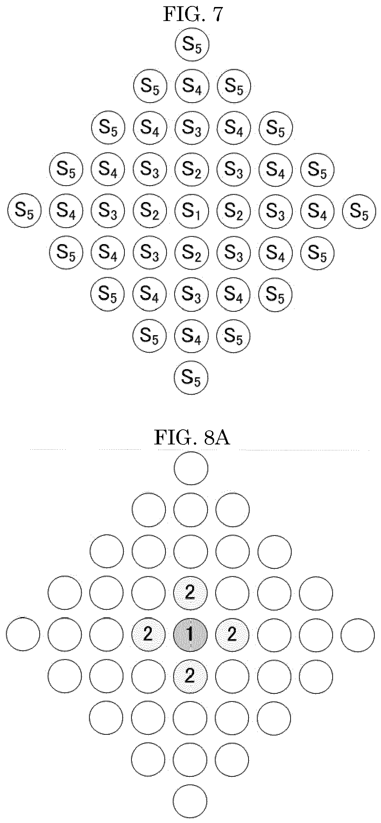

First, a collection of lattices within a radius r in a diamond lattice space is determined as a shell, and each lattice point is determined as $S_r$. Each lattice point $S_r$ is represented as in FIG. 7.

For example, collections $V_1$ to $V_5$ of lattice points to which amino acid residues of Nos. 1 to 5 are moved is represented as in FIGs. 8A to 8D.

In FIG. 8A, $V_1 = S_1$, and $V_2 = S_2$.

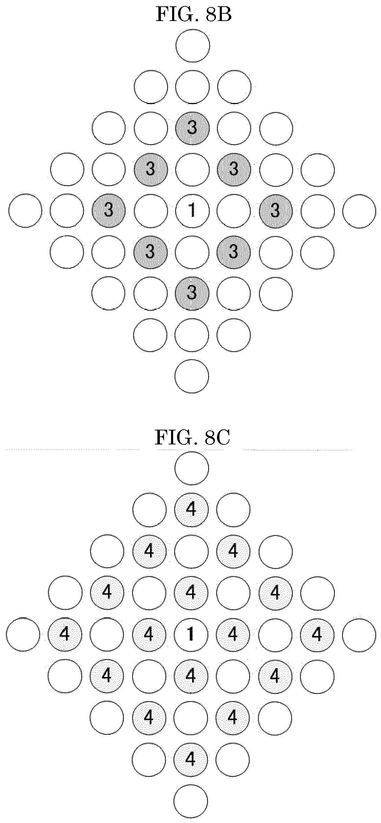

In FIG. 8B, $V_3 = S_3$.

In FIG. 8C, $V_4 = S_2 \ S_4$.

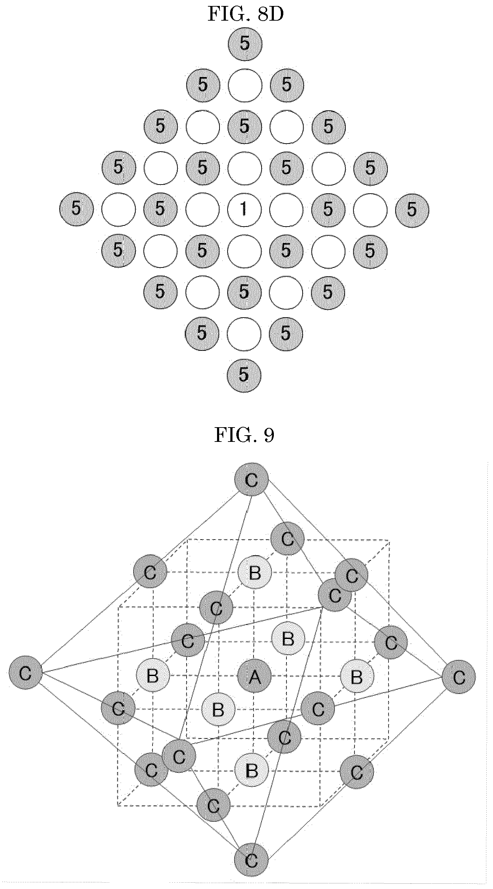

In FIG. 8D, $V_5 = S_3, S_5$.

Note that, when $S_1$, $S_2$, and $S_3$ are represented in three dimension, $S_1$, $S_2$, and $S_3$ represented as in FIG. 9. In FIG. 9, $A = S_1$, $B = S_2$, and $C = S_3$.

A space $V_i$ used for i-numbered amino acid residues in a protein having amino acid residues in the number of n is represented by the following formula.

$$V_i = \bigcup_{r \in J} S_r$$

In the formula above, i = {1, 2, 3, ......n}.

In case of an odd numbered (i = odd number) amino acid residue, J = {1, 3, .....i}. In case of an even numbered (i = even number) amino acid residue, J = {2, 4, .....i}.

<Step S102>

Next, whether the number (n) of amino acid residues is an even number or an odd number is judged. In the case where the number (n) thereof is an even number, the method proceeds to Step S104. In the case where the number (n) thereof is an odd number, the method proceeds to Step S103 (S102).

<Step S103>

Next, in the case where the number (n) of amino acid residues is an odd number, a linking group S and a terminal group E are added to the elements to be arranged in the three-dimensional lattice space in Step S103.

The linking group S is inserted between the amino acid residue arranged in the order of n and the amino acid residue arranged first in the cyclic protein. The terminal group E is bonded to the linking group S. A restriction is given to the terminal group E where the restriction is a restriction for arranging the terminal group E to face the amino acid residue arranged in the order of n with the linking group S being between the terminal group E and the amino acid residue arranged in the order of n.

<Step S104>

Next, a collection of lattice points which are locations to which the i-numbered amino acid residue, the linking group S, and the terminal group E move is determined as $V_i$ (S104).

As described above, a space to which an amino acid residue, a linking group S, and a terminal group E.

<Step S105>

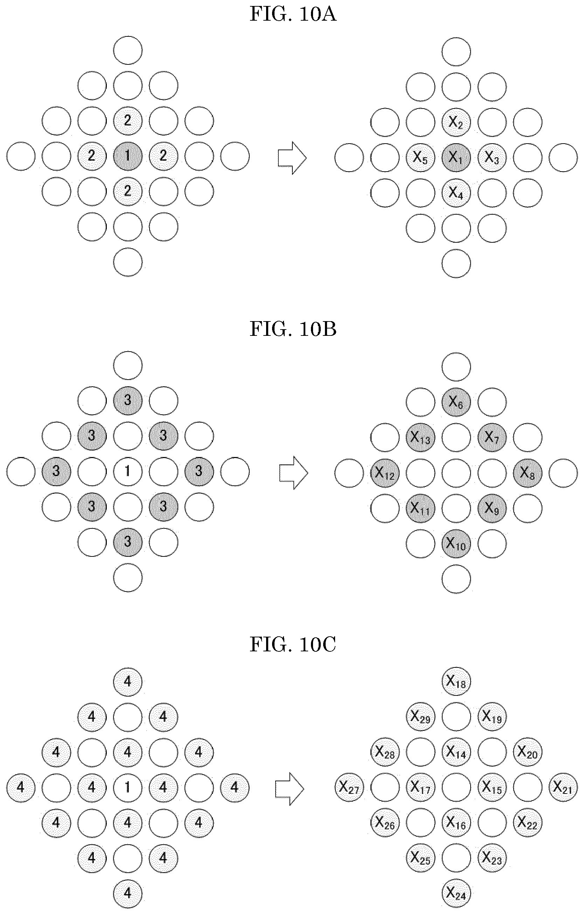

Next, a bit is assigned to each of the lattice points. Specifically, special information is assigned to each of bits $X_1$ to $X_n$ (S105). As illustrated in FIGs. 10A to 10C, specifically, a bit expressing presence of an amino acid residue, a linking group, or a terminal group in that position as 1 and absence of an amino acid residue, a linking group, or a terminal group as 0 is assigned with respect to a space to which each of amino acid residues is arranged. Note that, in FIGs. 10A to 10C, a plurality of $X_i$ are assigned to amino acid residues 2 to 4, but in reality one bit $X_i$ is assigned to one amino acid residue 1.

<Step S106>

Next, $H_{one}$, $H_{conn}$, $H_{olap}$, $H_{pair}$, $H_{bond}$, and $H_{end}$ are set and an Ising model obtained through conversion based on restriction conditions related to each lattice point is created (S106).
In the diamond encoding method, the entire energy can be expressed as follows.
The formula below is a formula of the entire energy when the number (n) of amino acid residues is an even number.

$$E(x) = H = H_{one} + H_{conn} + H_{olap} + H_{pair}$$

The formula below is a formation of the entire energy when the number (n) of amino acid residues is an odd number.

$$E(x) = H = H_{one} + H_{conn} + H_{olap} + H_{pair} + H_{bond} + H_{end}$$

In the formula above, $H_{one}$ is a restriction that there is only one from each of first to n-numbered amino acid residues.
$H_{conn}$ is a restriction that the first to n-numbered amino acid residues are all linked with one another.
$H_{olap}$ is a restriction that the first to n-numbered amino acid residues are not overlapped with one another.
$H_{pair}$ is a restriction representing an interaction between amino acid residues
$H_{bond}$ is a restriction representing that the linking group S is next to the first amino acid residue and the n-numbered amino acid residue.
$H_{end}$ is a restriction representing that the terminal group E faces to the n-numbered amino acid residues with the linking group S being between the terminal group E and the n-numbered amino acid residues.

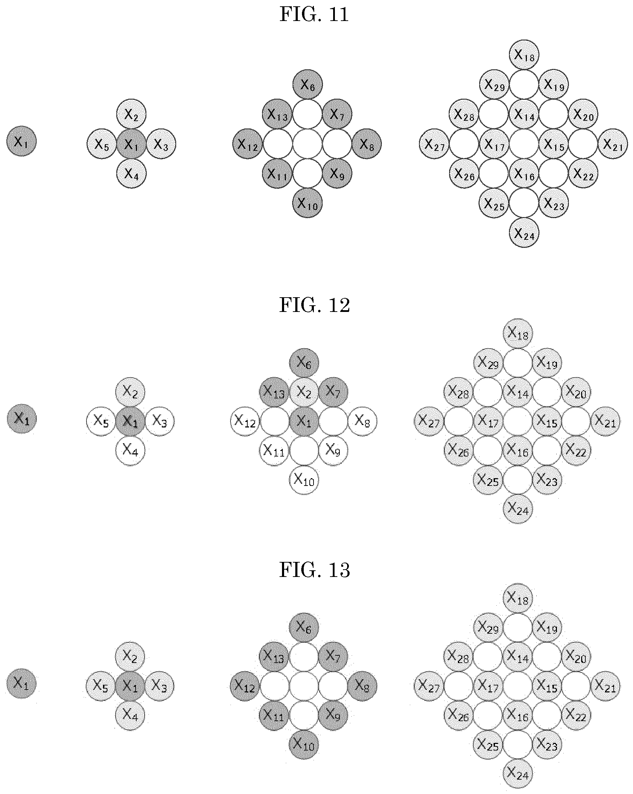

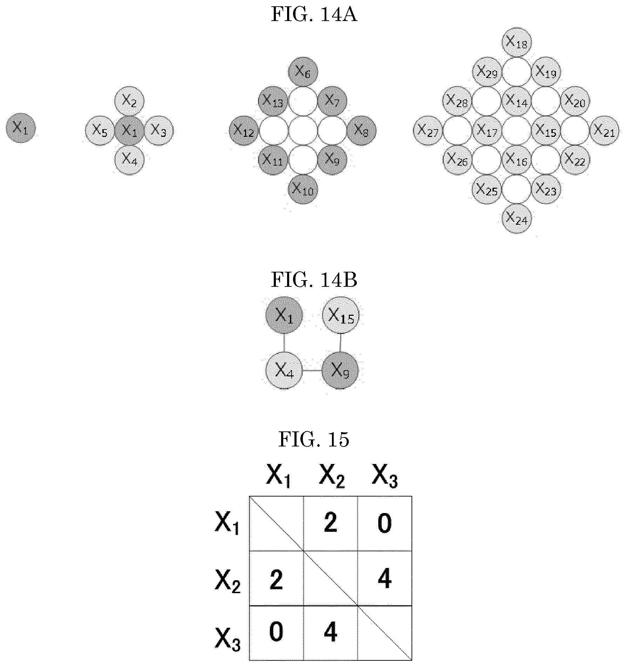

One example of each restriction is as follows.
Note that, in FIGs. 11 to 14A, and 14B described below, $X_1$ is a position to which an amino acid residue of No. 1 can be arranged.
$X_2$ to $X_5$ are positions to which an amino acid residue of No. 2 can be arranged.
$X_6$ to $X_{13}$ are positions to which an amino acid residue of No. 3 can be arranged.
$X_{14}$ to $X_{29}$ are positions to which an amino acid residue of No. 4 can be arranged.
One example of $H_{one}$ is presented below.

$$H_{one} = \lambda_{one} \sum_{i=0}^{N-1} \sum_{x_a, x_b, \in Q_i, a<b} x_a x_b$$

In the function above, $X_a$ and $X_b$ may be 1 or 0. Specifically, $H_{one}$ is a function that energy increases when any two or more of $X_2$, $X_3$, $X_4$, and $X_5$ are 1, because only one of $X_2$, $X_3$, $X_4$, and $X_5$ is 1 in FIG. 11, and is a term of penalty and becomes 0 when only one of $X_2$, $X_3$, $X_4$, and $X_5$ is 1.
Note that, in the function above, $\lambda_{one}$ is a weighting coefficient.
One example of $H_{conn}$ is presented below.

$$H_{conn} = \lambda_{conn} \left( N - 1 - \sum_{i=0}^{N-1} \sum_{x_d \in Q_i} \sum_{x_u \in \eta(x_d) \cap Q_{i+1}} x_d x_u \right)$$

In the function above, $X_d$ and $X_u$ may be 1 or 0. Specifically, $H_{conn}$ is a formula that energy decreases as long as $X_{13}$, $X_6$, or $X_7$ is 1 when $X_2$ is 1 in FIG. 12, and is a penalty term and becomes 0 when all of the amino acid residues are linked with one another.

Note that, in the function above, $\lambda_{conn}$ is a weighting coefficient. For example, the relationship of $\lambda_{one} > \lambda_{conn}$ is satisfied.

One example of $H_{olap}$ is presented below.

$$H_{olap} = \lambda_{olap} \sum_{v \in V} \sum_{x_a, x_b \in \theta(v), a < b} x_a x_b$$

In the function above, $X_a$ and $X_b$ are 1 or 0. Specifically, $H_{olap}$ is a term generating a penalty when $X_{14}$ is 1 with $X_2$ being 1 in FIG. 13.

Note that, in the function above, $\lambda_{olap}$ is a weighting coefficient.

One example of $H_{pair}$ is presented below.

$$H_{pair} = \frac{1}{2} \sum_{i=0}^{N-1} \sum_{x_a \in Q_i} \sum_{x_b \in \eta(x_a)} P_{\omega(x_a)\omega(x_b)} x_a x_b$$

In the function above, $X_a$ and $X_b$ may be 1 or 0. Specifically, $H_{pair}$ is a function that energy decreases due to interaction $P_{\omega(x1)\omega(x15)}$ between the amino acid residue of $X_1$ and the amino acid residue of $X_{15}$ when $X_{15}$ is 1 with $X_1$ being 1 in FIGs. 14A and 14B. The interaction $P_{\omega(x1)\omega(x15)}$ is determined by a combination of two amino acid residues. For example, the interaction $P_{\omega(x1)\omega(x15)}$ is determined with reference to Miyazawa-Jernigan (MJ) matrix.

$H_{bond}$ is appropriately set to satisfy a restriction that the linking group S is next to the first amino acid residue and the n-numbered amino acid residue.

$H_{end}$ is appropriately set to satisfy a restriction that the terminal group E faces the n-numbered amino acid residue with the linking group S being between the terminal group E and the n-numbered amino acid residue.

Then, H is calculated by synthesizing $H_{one}$, $H_{conn}$, $H_{olap}$, and $H_{pair}$, and optionally $H_{bond}$, and $H_{end}$.

Next, a weighting coefficient ($\lambda_{one}$, $\lambda_{conn}$, and $\lambda_{olap}$) of each functions above is extracted.

Next, a weight file corresponding to the extracted weight coefficient is created. For example, the weight file is a matrix. In case of $2X_1X_2 + 4X_2X_3$, for example, the weight file is a matrix file as illustrated in FIG. 15.

The following energy formula of the Ising model can be expressed by using the created weight file.

$$E(x) = -\sum_{\langle i,j \rangle} W_{ij} x_i x_j - \sum_i b_i x_i$$

In the function above, the states $X_i$ and $X_j$ may be 0 or 1, where 0 means absence and 1 means presence. $W_{ij}$ that is a first term of the right side is a weighting coefficient.

The first term of the right side is the integration of the product of the state of two neuron circuits and the weighting value for all selectable combinations of two neuron circuits from the whole neuron circuits without any omission or overlap.

Moreover, the second term of the right side is the integration of the product of the bias value and state of each of the whole neuron circuits. $b_i$ is a bias value of the i-numbered neuron circuit.

<Step S107>

Next, the annealing machine executes a ground state search of the Ising model converted based on the restriction conditions related to each of the lattice points according to simulated annealing to thereby calculate the minimum energy of the Ising model (S107).

The annealing machine may be any of a quantum annealing machine, a semiconductor annealing machine using a semiconductor technology, or simulated annealing executed by software using a central processing unit (CPU) or a graphics processing unit (GPU), if the computer for use is a computer employing an annealing system for performing a ground state search of an energy function represented by the Ising model.

One example of simulated annealing and the annealing machine will be described below.

Simulated annealing (SA) is a kind of Monte Carlo methods, and a method for stochastically determining using a random numerical value. In the description below, a problem for minimizing a value of an evaluation function to be optimized is taken as an example, and the value of the evaluation function is called energy. In case of maximization, a plus or minus sign of the evaluation function may be changed.

Starting with an initial state where one discrete value is assigned to each variable, a state that is close to the initial state (e.g., a state where only one variable is changed) is selected from the current state (combinations of values of the variables), and then state transition thereof is studied. An energy change for the state transition is calculated, and whether the state transition is adapted to change the state or the original state is retained without adapting the state transition is determined stochastically depending on the calculated value. When the adaption probability of a case where energy reduces is selected to be larger than the adaption probability of a case where energy increases, the state change occurs in the tendency that the energy reduces on average, and it is expected that the state transits to an appropriate state over time. Then, ultimately, it is possible to obtain an approximation solution that gives energy close to an optimum solution or an optimum value. If the case where energy reduces is adopted deterministically and the case where energy increases is not adapted, the energy change is in the state of weakly decreasing with respect to time, but the change will stop once the local solution is reached. Since there are a large number of local solutions in the discrete optimization problem as described above, it is most likely that the state is trapped by a local solution that is not very close to an optimum value. Accordingly, it is important to stochastically determine whether to adapt.

It is proved in the simulated annealing that the state reaches the optimum solution with the limit of infinite time (the number of iteration) when the adaptation (tolerance) probability of the state transition is determined as follows.

(1) With respect to an energy change (energy decrease) value ($-\Delta E$) along with the state transition, acceptance probability p of the state transition is determined by any of the following functions f ().

$$p(\Delta E, T) = f(-\Delta E / T) \qquad \text{(Formula 1-1)}$$

$$f_{metro}(x) = \min(1, e^x) \quad \text{(Metropolis method)} \quad \text{(Formula 1-2)}$$

$$f_{Gibbs}(x) = \frac{1}{1 + e^{-x}} \qquad \text{(Gibbs method)} \qquad \text{(Formula 1-3)}$$

In the formula above, T is a parameter called a temperature value, which is changed as follows.
(2) The temperature value T is logarithmically decreased relative to the number of iteration t as represented by the following formula.

$$T = \frac{T_0 \log(c)}{\log(t + c)} \qquad \text{(Formula 2)}$$

In the formula above, $T_0$ is an initial temperature value, and is desired to be sufficiently large depending on a problem.

In the case where acceptance probability represented by the formula of (1) is used, once the state reaches a steady state after sufficient iterations, occupancy probability of each state follows the Boltzmann distribution for a thermal equilibrium state in thermodynamics.

As the temperature is gradually lowered from a high temperature, occupancy probability of a low energy state increases. Therefore, a low energy state is supposed to be obtained when the temperature is sufficiently reduced. The state as described above is very similar to a state change occurred when a material is developed. Therefore, the method described above is called simulated annealing. The stochastic occurrence of the state transition of energy increase is equivalent to thermal excitation in physics.

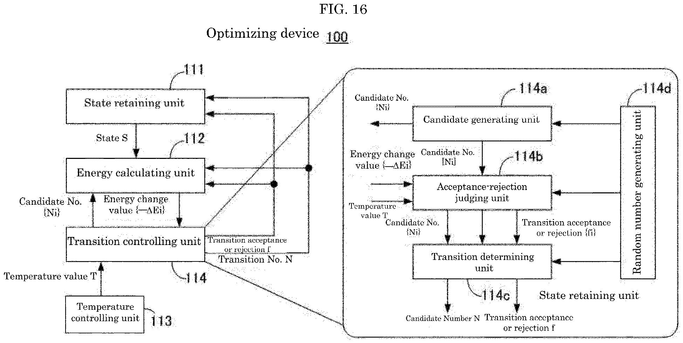

An optimizing device (arithmetic unit 18) for performing simulated annealing is illustrated in FIG. 16. The descriptions below include a case where a plurality of candidates of state transitions are generated, but a transition candidate is generated one by one in the original basic simulated annealing.

An optimizing device 100 includes a state retaining unit 111 configured to retain a current state S (a plurality of state variable values). Moreover, the optimizing device 100 includes an energy calculating unit 112 configured to calculate an energy change value {-ΔEi} of each state transition when a state transition occurs from the current state S due to a change of any of the state variable values. Moreover, the optimizing device 100 includes a temperature controlling unit 113 configured to control a temperature value T and a transition controlling unit 114 configured to control a state transition.

The transition controlling unit 114 is configured to stochastically determine whether any of the state transitions is adapted or not according to the correlation between the energy change value {-ΔEi} and the thermal excitation energy based on the temperature value T, the energy change value {-ΔEi}, and the random numerical value.

The transition controlling unit 114 is further subdivided. The transition controlling unit 114 includes a candidate generating unit 114a configured to generate candidates of state transition, and a judging unit 114b configured to stochastically judge on each candidate whether the state transition is allowed or not based on the energy change value {-ΔEi} and temperature value T thereof. The transition controlling unit 114 further includes a transition determining unit 114c configured to determine the candidate to be adapted among the allowed candidates, and a random number generating unit 114d configured to generate probability variables.

An operation of one iteration is as follows. First, the candidate generating unit 114a generates one or more candidates (candidate number {Ni}) of state transition from the current state S retained in the state retaining unit 111 to the next state. The energy calculating unit 112 calculates an energy change value {-ΔEi} for each of state transitions listed as candidates using the current state S and the candidates of state transition. The judging unit 114b accepts the state transition with the acceptance probability of the formula of (1) above according to the energy change value {-ΔEi} of each state transition using the temperature value T generated by the temperature controlling unit 113 and the probability variable (random numerical value) generated by the random number generating unit 114d. Then, the judging unit 114b outputs acceptance or rejection {fi} of each state transition. In the case where there are a plurality of the accepted state transitions, the transition determining unit 114c randomly selects one of the accepted state transitions using the random numerical value. The transition determining unit 114c outputs the transition number N of the selected state transition and acceptance or rejection of the transition f. In the case where there is the accepted state transition, the value of the state variable stored in the state retaining unit 111 is updated according to the adapted state transition.

The iteration described above is started from an initial state and repeated with decreasing the temperature value by the temperature controlling unit 113. When the finishing judgement conditions, such as reaching the certain number of iterations, or the energy being dropped below a certain value, are satisfied, the operation is completed. The answer output by the optimizing device 110 is the state at the time of the finish.

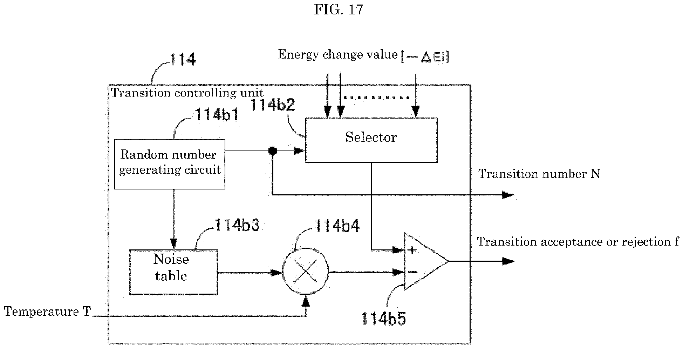

FIG. 17 is a block diagram of a circuit level of a structural example of arithmetic part used for a transition controlling unit, particularly a judging unit, in typical simulated annealing where a candidate is generated one by one.

A transition controlling unit 114 includes a random number generator 114b1, a selector 114b2, a noise table 114b3, a multiplier 114b4, and a comparator 114b5.

The selector 114b2 is configured to select the value corresponding to the transition number N that is a random numerical value generated by the random number generator 114b1 among the energy change values {-ΔEi} calculated for candidates of each state transition, and then output the value.

Functions of the noise table 114b3 will be described later. As the noise table 114b3, for example, a memory, such as a random access memory (RAM), and a flash memory, can be used.

The multiplier 114b4 outputs a product (corresponding to the above-described thermal excitation energy) obtained by multiplying the value output by the noise table 114b3 with the temperature value T.

The comparator 114b5 outputs, as transition acceptance or rejection f, a comparison result obtained by comparing the product result output by the multiplier 114b4 and the energy change value -ΔE selected by the selector 114b2.

The transition controlling unit 114 illustrated in FIG. 17 basically has the above-mentioned functions as they are, but a mechanism for accepting state transition with the acceptance probability represented by the formula (1) has not yet been described. Therefore, the mechanism will be supplementary described.

The circuit that outputs 1 with the acceptance probability p and 0 with (1-p) has two inputs A and B, can be realized by inputting the acceptance probability p to the input A of the comparator and a uniform random number having the value in the interval [0, 1) to the input B of the comparator where the comparator outputs 1 when A > B and outputs 0 when A < B. Accordingly, the above-described function can be realized by inputting the value of the acceptance probability p calculated from the energy change value and the temperature value T using the formula of (1) to the input A of the comparator.

Specifically, the above-described function can be realized with the circuit that outputs 1 when f($\Delta$E/T) is larger than u, where f is the function represented by the formula of (1) and u is a uniform random number having the value of the interval [0, 1).

The circuit may be as it is, but the same function can be also realized by performing the following deformation. The magnitude relationship of two numbers does not change when the same monotone increasing function is given the two numbers. Therefore, output does not change even when the same monotone increasing function is gives two inputs of the comparator. It can be understood that a circuit outputting 1 when -$\Delta$E/T is larger than $f^{-1}$(u) is acceptable when an inverse function $f^{-1}$ of f is used as the monotone increasing function. Since the temperature value T is a positive value, moreover, a circuit outputting 1 when -$\Delta$E is larger than $Tf^{-1}$(u) is acceptable. The noise table 114b3 in FIG. 17 is a conversion table for realizing the inverse function $f^{-1}$(u), and a table for outputting a value of the following function with respect to an input of discretized interval [0, 1).

$$f_{metro}^{-1}(u) = \log(u) \qquad \text{(Formula 3-1)}$$

$$f_{Gibbs}^{-1}(u) = \log\left(\frac{u}{1-u}\right) \qquad \text{(Formula 3-2)}$$

The transition controlling unit 114 also includes a latch configured to retain judgement results etc., a state machine configured to generate timing thereof, etc., but the above-mentioned units are omitted in FIG. 17 in order to simplify the illustration.

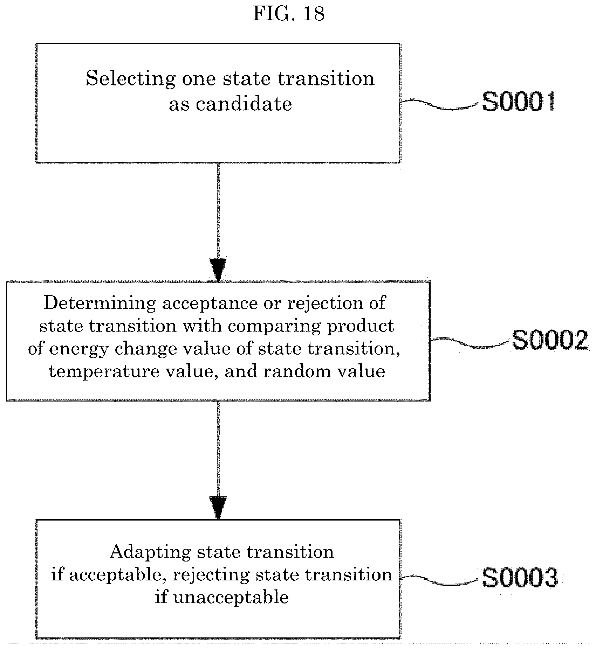

FIG. 18 illustrates an operation flow of the transition controlling unit 114. The operation flow includes a step for selecting one state transition as a candidate (S0001), a step for determining acceptance or rejection of the state transition with comparing a product of the energy change value of the state transition, temperature value, and random numerical value (S0002), and a step for adapting the state transition if the state transition is acceptable and rejecting if the state transition is not acceptable (S0003).

<Step S108>

The calculation result is output in Step S108. The result may be output as a three-dimensional structure diagram of a protein or as coordinate information of each amino acid residue constituting a protein.

(Program)

The disclosed program is a program for executing the disclosed method for searching a structure of a cyclic molecule.

Preferable embodiments of the program for executing the method for searching a structure of a cyclic molecule are identical to preferable embodiments of the disclosed method for searching a structure of a cyclic molecule.

The program can be created using any of various programing languages known in the art according to a configuration of a computer system for use, a type or version of an operation system for use.

The program may be recorded on storage media, such as an integral hard disk, and an external hard disk, or recorded on a storage medium, such as a compact disc read only memory (CD-ROM), a digital versatile disk read only memory (DVD-ROM), a magneto-optical (MO) disk, and a universal serial bus (USB) memory stick (USB flash drive). In the case where the program is recorded on a storage medium, such as a CD-ROM, a DVD-ROM, an MO disk, and an USB memory stick, the program can be used, as required, directly or by installing a hard disk via a storage medium reader equipped in a computer system. Moreover, the program may be recorded in an external memory region (e.g. another computer) accessible from the computer system via an information and communication network, and the program may be used, as required, by directly from the external memory region or installing into a hard disk from the external memory region via the information and communication network.

The program may be divided into predetermined processes and recorded on a plurality of recording media.

(Recording medium)

The disclosed recording medium is a recording medium having stored therein the disclosed program.

The disclosed recording medium can be read by a computer.

The disclosed recording medium may be a transitory recording medium or a non-transitory recording medium.

The recording medium is not particularly limited and may be appropriately selected depending on the intended purpose. Examples of the recording medium include integral hard disks, external hard disks, CD-ROMs, DVD-ROMs, MO disks, and USB memory sticks.

The recording medium may be a plurality of recording media to which predetermined processes divided from the program are recorded.

(Device for searching structure of cyclic molecule)

The disclosed device for searching a structure of a cyclic molecule includes at least a creating unit, and may further include other units, such as a calculating unit.

The creating unit is configured to arrange compounds groups in the number of n to lattice points of a three-dimensional lattice space that is a collection of lattices to create a three-dimensional structure of a cyclic molecule in the three-dimensional lattice space.

In the case where the number (n) of the compound groups of the cyclic molecule is an odd number, the creating unit is configured to insert a linking group between the compound group arranged in the order of n and the compound group arranged first within the cyclic molecule, arrange the linking group on a lattice point, and adjust the arrangement in a manner that the compound group arranged in the order of n and the compound group arranged first do not face each other with the linking group being between the compound arranged in the order of n and the compound group arranged first.

The calculation unit is configured to perform a ground state search on the created three-dimensional structure of the cyclic molecule using simulated annealing to calculate minimum energy. Specifically, for example, the calculation of the minimum energy can be performed by the method described in Step S106 and Step S107.

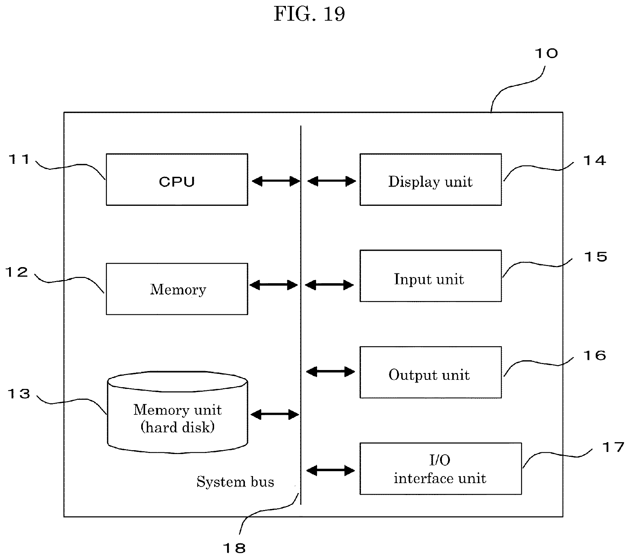

FIG. 19 illustrates a structural example of the disclosed device for searching a structure of a cyclic molecule.

For example, the device for searching a structure of a cyclic molecule 10 is composed by connecting CPU 11, a memory 12, a memory unit 13, a display unit 14, an input unit 15, an output unit 16, and an I/O interface unit 17 via a system bus 18.

The central processing unit (CPU) 11 is configured to perform calculations (e.g., four arithmetic operations, and relational operations), and control of operations of hardware and software.

The memory 12 is a memory, such as a random access memory (RAM), and a read only memory (ROM). The RAM is configured to store an operating system (OS) and application programs read from the ROM and the memory unit 13, and function as a main memory and work area of the CPU 11.

The memory unit 13 is a device for storing various programs and data. For example, the memory unit 13 is a hard disk. In the memory unit 13, programs to be executed by the CPU 11, data for executing the programs, and an OS are stored.

The program is stored in the memory unit 13, loaded on the RAM (a main memory) of the memory 12, and executed by the CPU 11.

The display unit 14 is a display device. For example, the display unit is a display device, such as a CRT monitor, and a liquid crystal panel.

The input unit 15 is an input device for various types of data. Examples of the input unit include a key board, and a pointing device (e.g., a mouse).

The output unit 16 is an output device for various types of data. For example, the output unit is a printer.

The I/O interface unit 17 is an interface for connecting to various external devices. For example, the I/O interface unit enables input and output of data of CD-ROMs, DVD-ROMs, MO disks, and USB memory sticks.

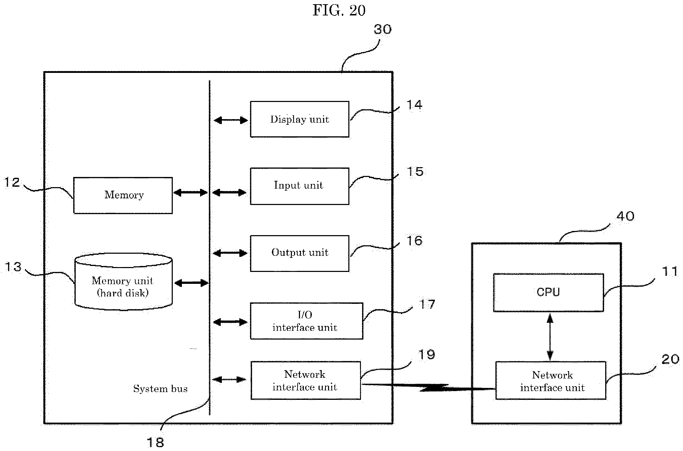

FIG. 20 illustrates another structural example of the disclosed device for searching a structure of a cyclic molecule.

The structural example of FIG. 20 is a structural example of a cloud-type calculation device, where CPU 11 is independent of a memory unit 13 etc. In the structural example, a computer 30 having stored therein the memory unit 13 and a computer 40 having stored therein the CPU 11 are coupled with each other via network interface units 19 and 20.

The network interface units 19 and 20 are hardware configured to communicate using Internet.

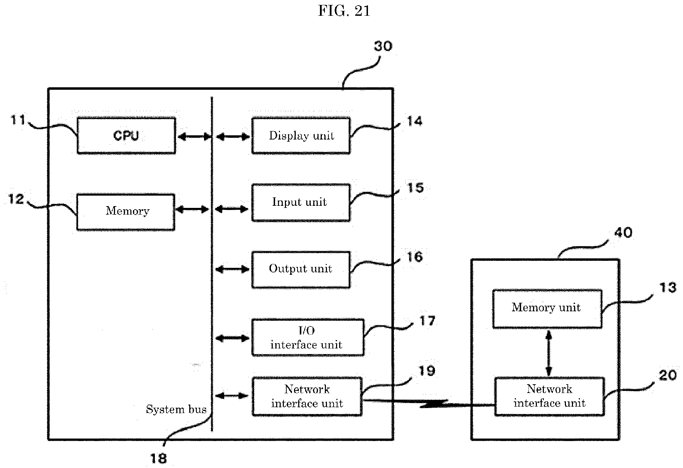

FIG. 21 illustrates another structural example of the disclosed device for searching a structure of a cyclic molecule.

The structural example of FIG. 21 is a structural example of a cloud-type calculation device, where a memory unit 13 is independent of CPU 11, etc. In the structural example, a computer 30 having stored therein the CPU 11 and a computer 40 having stored therein the memory unit 13 are coupled with each other via network interface units 19 and 20.

**Claims**

1. A method for searching a structure of a cyclic molecule, the method comprising:

   arranging each of compound groups in the number of n on each of lattice points to create a three-dimensional structure of the cyclic molecule in a three-dimensional lattice space, where the lattice points are lattice points of the three-dimensional lattice space that is a collection of lattices,
   wherein the method is a method for searching a stable structure of the cyclic molecule, in which the compound groups in the number of n are linked to form a ring, using a computer, and
   wherein in a case where the number (n) of the compound groups of the cyclic compound is an odd number, the arranging includes:
   inserting a linking group between the compound group arranged in the order of n and the compound group arranged first within the cyclic molecule, arranging the linking group on a lattice point, and adjusting the arrangement in a manner that the compound group arranged in the order of n and the compound group arranged first do not face each other with the linking group being between the compound arranged in the order of n and the compound group arranged first.

2. The method according to claim 1,
   wherein the adjusting the arrangement is performed by bonding a terminal group to the linking group to arrange the terminal group to face the compound group arranged in the order of n in the three-dimensional lattice space with the linking group being between the terminal group and the compound group.

3. The method according to claim 1 or 2, further comprising:
   performing a ground state search on the created three-dimensional structure of the cyclic molecule using simulated annealing to calculate minimum energy.

4. The method according to any of claims 1 to 3,
   wherein the arranging includes judging whether the number (n) of the compound groups of the cyclic molecule is an odd number or an even number.

5. The method according to any of claims 1 to 4,
   wherein the cyclic molecule is a cyclic protein.

6. The method according to claim 5,
   wherein the compound groups are amino acid residues.

7. A device for searching a structure of a cyclic molecule, the device comprising:

   a creating unit configured to arrange each of compounds in the number of n on each of lattice points to create a three-dimensional structure of the cyclic molecule in a three-dimensional lattice space, where the lattice points are lattice points of the three-dimensional lattice space that is a collection of lattices,
   wherein the device is a device for searching a stable structure of the cyclic molecule in which the compound groups in the number of n are linked to form a ring, and
   wherein in a case where the number (n) of the compound groups of the cyclic compound is an odd number, the creating unit is configured to insert a linking group between the compound group arranged in the order of n and the compound group arranged first within the cyclic molecule, arrange the linking group on a lattice point, and adjust the arrangement in a manner that the compound group arranged in the order of n and the compound group arranged first do not face each other with the linking group being between the compound arranged in the order of n and the compound group arranged first.

8. The device according to claim 7,
   wherein the creating unit is configured to execute the arrangement by bonding a terminal group to the linking group to arrange the terminal group to face the compound group arranged in the order of n in the three-dimensional lattice space with the linking group being between the terminal group and the compound group.

9. The device according to claim 7 or 8, further comprising:
a calculating unit configured to perform a ground state search on the created three-dimensional structure of the cyclic molecule using simulated annealing to calculate minimum energy.

10. The device according to any of claims 7 to 9,
wherein the creating unit is configured to judge whether the number (n) of the compound groups of the cyclic molecule is an odd number or an even number.

11. A program for causing a computer to execute a method for searching a structure of cyclic molecule, the method comprising:

   arranging each of compound groups in the number of n on each of lattice points to create a three-dimensional structure of the cyclic molecule in a three-dimensional lattice space, where the lattice points are lattice points of the three-dimensional lattice space that is a collection of lattices,
   wherein the program is a program for searching a stable structure of the cyclic molecule in which the compound groups in the number of n are linked to form a ring, and
   wherein in a case where the number (n) of the compound groups of the cyclic compound is an odd number, the arranging includes:
   inserting a linking group between the compound group arranged in the order of n and the compound group arranged first within the cyclic molecule, arranging the linking group on a lattice point, and adjusting the arrangement in a manner that the compound group arranged in the order of n and the compound group arranged first do not face each other with the linking group being between the compound arranged in the order of n and the compound group arranged first.

12. The program according to claim 13,
wherein the adjusting the arrangement is performed by bonding a terminal group to the linking group to arrange the terminal group to face the compound group arranged in the order of n in the three-dimensional lattice space with the linking group being between the terminal group and the compound group.

13. The program according to claim 11 or 12,
wherein the program causes the computer to execute a ground state search on the created three-dimensional structure of the cyclic molecule using simulated annealing to calculate minimum energy.

14. The program according to any of claims 11 to 13,
wherein the arranging includes judging whether the number (n) of the compound groups of the cyclic molecule is an odd number or an even number.

FIG. 1A

FIG. 1B

FIG. 1C

FIG. 2A

FIG. 2B

FIG. 2C

FIG. 2D

FIG. 2E

FIG. 3A

FIG. 3B

FIG. 3C

FIG. 4

FIG. 5

FIG. 6

S101 — Defining three-dimensional lattice space

S102 — Is the number (n) of amino acid residues odd number?  →No

S103 — Adding linking group S and terminal group E

Yes

S104 — Determining collection of lattice points that are locations No. i amino acid residue moves as $V_i$

S105 — Assigning information of space to $X_1$ to $X_n$

S106 — Creating Ising model

S107 — Determining the minimum value of E(x) by annealing machine

S108 — Outputting result

## FIG. 7

## FIG. 8A

FIG. 8B

FIG. 8C

FIG. 8D

FIG. 9

FIG. 10A

FIG. 10B

FIG. 10C

FIG. 11

FIG. 12

FIG. 13

FIG. 14A

FIG. 14B

FIG. 15

|       | $X_1$ | $X_2$ | $X_3$ |
|-------|-------|-------|-------|
| $X_1$ |       | 2     | 0     |
| $X_2$ | 2     |       | 4     |
| $X_3$ | 0     | 4     |       |

FIG. 16

Optimizing device 100

FIG. 17

Energy change value {— ΔEi}

114

Transition controlling unit

114b1    114b2

Random number
generating circuit

Selector

Transition number N

114b3    114b4

Noise
table

Transition acceptance or rejection f

Temperature T

114b5

FIG. 18

Selecting one state transition
as candidate
S0001

Determining acceptance or rejection of
state transition with comparing product
of energy change value of state transition,
temperature value, and random value
S0002

Adapting state transition
if acceptable, rejecting state transition
if unacceptable
S0003

FIG. 19

10

11 — CPU ⟷ ⟷ Display unit — 14

12 — Memory ⟷ ⟷ Input unit — 15

⟷ Output unit — 16

13 — Memory unit (hard disk) ⟷

System bus

I/O interface unit — 17

18

FIG. 20

30

| | | |
|---|---|---|
| | Display unit | 14 |
| Memory 12 | Input unit | 15 |
| | Output unit | 16 |
| Memory unit (hard disk) 13 | I/O interface unit | 17 |
| System bus | Network interface unit | 19 |

18

40

CPU 11

Network interface unit 20

FIG. 21

**EUROPEAN SEARCH REPORT**

Europäisches Patentamt
European Patent Office
Office européen des brevets

Application Number

EP 19 20 5295

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Ryan Babbush ET AL: "Construction of Energy Functions for Lattice Heteropolymer Models: Efficient Encodings for Constraint Satisfaction Programming and Quantum Annealing" In: "Advances in Chemical Physics: Volume 155", 11 April 2014 (2014-04-11), John Wiley & Sons, Inc., Hoboken, New Jersey, XP055692281, ISBN: 978-1-118-75577-8 pages 201-244, DOI: 10.1002/9781118755815.ch05, * the whole document * | 1-14 | INV. G16C20/40 G16B15/00 |
| A | WO 2018/154789 A1 (FUJITSU LTD [JP]) 30 August 2018 (2018-08-30) * the whole document * | 1-14 | |
| A | ANWAR RAYAN ET AL: "Exploring the conformational space of cyclic peptides by a stochastic search method", JOURNAL OF MOLECULAR GRAPHICS AND MODELLING, vol. 22, no. 5, 1 May 2004 (2004-05-01), pages 319-333, XP055692382, US ISSN: 1093-3263, DOI: 10.1016/j.jmgm.2003.12.012 * the whole document * | 1-14 | TECHNICAL FIELDS SEARCHED (IPC) G16C G16B |

-/--

The present search report has been drawn up for all claims

| Place of search | Date of completion of the search | Examiner |
|---|---|---|
| The Hague | 8 May 2020 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 20 5295

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | IVAN DOTU ET AL: "On Lattice Protein Structure Prediction Revisited", IEEE/ACM TRANSACTIONS ON COMPUTATIONAL BIOLOGY AND BIOINFORMATICS, IEEE SERVICE CENTER, NEW YORK, NY, US, vol. 8, no. 6, 1 November 2011 (2011-11-01), pages 1620-1632, XP058005205, ISSN: 1545-5963, DOI: 10.1109/TCBB.2011.41 * the whole document * ----- | 1-14 | |
| A | HITOSHI GOTO ET AL: "An efficient algorithm for searching low-energy conformers of cyclic and acyclic molecules", JOURNAL OF THE CHEMICAL SOCIETY, PERKIN TRANSACTIONS 2: PHYSICAL ORGANIC CHEMISTRY, no. 2, 1 January 1993 (1993-01-01), pages 187-198, XP055692597, GB ISSN: 0300-9580, DOI: 10.1039/P29930000187 * the whole document * ----- | 1-14 | |
| A | HONGTAO YU ET AL: "Toward structure prediction of cyclic peptides", PHYSICAL CHEMISTRY CHEMICAL PHYSICS, vol. 17, no. 6, 5 December 2014 (2014-12-05), pages 4210-4219, XP055692663, ISSN: 1463-9076, DOI: 10.1039/C4CP04580G * the whole document * ----- | 1-14 | TECHNICAL FIELDS SEARCHED (IPC) |

The present search report has been drawn up for all claims

| Place of search | Date of completion of the search | Examiner |
|---|---|---|
| The Hague | 8 May 2020 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 19 20 5295

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2020

| Patent document cited in search report | Publication date | Patent family member(s) | Publication date |
|---|---|---|---|
| WO 2018154789 A1 | 30-08-2018 | EP 3588335 A1 | 01-01-2020 |
| | | JP WO2018154789 A1 | 12-12-2019 |
| | | US 2019362815 A1 | 28-11-2019 |
| | | WO 2018154789 A1 | 30-08-2018 |

EPO FORM P0459

For more details about this annex : see Official Journal of the European Patent Office, No. 12/82